# EUROPEAN PATENT APPLICATION

(11) **EP 4 600 354 A1**
(43) Date of publication of application: **13.08.2025**
(21) Application number: 24156900.3
(22) Date of filing: 09.02.2024
(51) Int. Cl.: C12N 9/02, C12N 9/10, C12N 9/22, C12N 15/82

(54) **PLANT CELL AND PLANT HAVING REDUCED CONTENT OF STEROIDAL GLYCOALKALOIDS AND METHODS THEREFOR**

(71) Applicant: Sveriges Stärkelseproducenters Förening Upa, 291 91 Kristianstad (SE)
(72) Inventor: SITBON, Folke, 752 18 Uppsala (SE); HOFVANDER, Per, 237 41 Bjärred (SE); ANDERSSON, Mariette, 222 70 Lund (SE); MERINO SIERRA, Irene Maria, 752 21 Uppsala (SE); LIU, Ying, 227 29 Lund (SE)
(74) Representative: Brann AB

(57) **Abstract**

A method of obtaining a genetically modified plant cell having reduced content of at least one steroidal glycoalkaloid, wherein the method comprises the steps of: i. providing a plant cell, and ii. modifying the genome of the plant cell so as to reduce or inhibit expression of at least one gene selected from the group consisting of *SMO1-L, DWF7-L,* and *TAM iso 2.* A genetically modified plant cell and a genetically modified plant, as well as a method of obtaining a genetically modified plant and uses of a genetically modified plant in a process for producing starch or in a process of producing a food product are also provided.

## Description

### TECHNICAL FIELD

The technology proposed herein relates generally to the field of methods for genetically modified plants. More particularly, the technology proposed herein relates to a plant cell and plant, in particular potato, having reduced content of at least one steroidal glycoalkaloid and methods for obtaining the plant cell and plant.

### BACKGROUND

Potato (*Solanum tuberosum* L.) is the fourth most important food crop species in the world after maize (Zea *mays* L.), wheat (*Triticum aestivum*), and rice (*Oryza sativa*)*.* Potato is not only a staple food but also a versatile raw material for various industrial applications such as starch-related products, animal feed and raw material for biofuel production.

Steroidal alkaloids (SAs) and their glycosylated forms, known as steroidal glycoalkaloids (SGAs), are nitrogen-containing secondary metabolites identified in various potato species. In cultivated potatoes, α-chaconine and α-solanine are the predominant SGAs, constituting over 95% of the total SGA content; while wild potato species exhibit a broader SGA composition, including minor SGAs like solamarine, solamargine, solasonine, demissine and leptine. Although the concentration of SGAs in potato tubers is relatively low compared to other plant parts, such as flowers, sprouts and leaves; there is a nonuniform distribution in the tubers, with the highest levels in peels, especially around the sprout eyes of the outer layer, and lower levels in the fresh inner tissues. The SGA levels in potato tubers can significantly increase upon various abiotic and biotic stresses during growing and storage, such as drought, heat, wounding and light exposure. Despite their crucial role in plant defense mechanisms, SGAs are often regarded as natural toxins with potential adverse effects on human health. Thus, a limit amount of 200 mg total SGA kg-1 fresh weight (FW) is widely recommended in potato tubers for food safety consideration.

During potato starch production, substantial quantities of by-products, including potato fruit juice (PFJ) and potato pulp, are generated, that either need to be disposed of or transformed into useful side stream products. PFJ is rich in economically valuable compounds, e.g. amino acids, minerals, and proteins (ca. 2%). Potato proteins stand out for their superior quality, marked by a high lysine content, compared to proteins from cereal sources. Potato protein, with the properties of low allergenicity, high nutritional value, have the potential to substitute other plant-based and animal-based proteins in food production. Potato pulp primarily comprises tuber cell wall materials and is abundant in pectin, presenting a potential source for manufacture high-value potato dietary fiber. However, the presence of SGAs poses a significant challenge, given their concentration in residual by-products, complicating the extraction of proteins and fibers without compromising their functional properties. Various techniques for extraction, such as acidic precipitation and thermal coagulation, have been explored during purification and functional recovery of potato proteins from PFJ. Yet, the functionality of proteins isolated via thermal treatment might be compromised, limiting their application to animal feed.

As shown in the foregoing, there is accordingly a need for further approaches to managing and/or mitigating the adverse effects of SGA in plants such as potato.

### OBJECTS OF THE TECHNOLOGY PROPOSED HEREIN

It is accordingly a first object of the technology proposed herein to provide a method of obtaining a genetically modified plant cell.

It is a further object of the technology proposed herein to provide a genetically modified plant cell.

It is yet a further object of the technology proposed herein to provide a method of obtaining a genetically modified plant.

It is yet a further object of the technology proposed herein to provide genetically modified plant.

It is yet a further object of the technology proposed herein to provide a use of a genetically modified plant or part thereof in a process for producing starch.

It is yet a further object of the technology proposed herein to provide a use of a genetically modified plant or part thereof in a process for producing a food product.

### SUMMARY

At least one of the abovementioned objects, or at least one of the further objects which will become evident from the below description, are according to corresponding first, second, third, and fourth aspects of the technology proposed herein obtained by a method of obtaining a genetically modified plant cell having reduced content of at least one steroidal glycoalkaloid, wherein the method comprises the steps of:
i. providing a plant cell, and
ii. modifying the genome of the plant cell so as to reduce or inhibit expression of at least one gene selected from the group consisting of *SMO1-L, DWF7-L,* and *TAM iso 2,*
   and

a genetically modified plant cell having reduced content of at least one steroidal glycoalkaloid, wherein the genome of the plant cell has been modified so as to reduce or inhibit expression of at least one gene selected from the group consisting of *SMO1-L, DWF7-L,* and *TAM iso 2,*
   and
a method of obtaining a genetically modified plant, comprising the steps of:
   i. providing a genetically modified plant cell obtained by the method according to the first aspect of the technology proposed herein or according to the second aspect of the technology proposed herein, and
   ii. cultivating or regenerating the genetically modified plant cell to obtain the genetically modified plant,
      and
a genetically modified plant comprising or consisting of:
   - one or more plant cells obtained by the method according to the first aspect of the technology proposed herein, or
   - one or more plant cells according to the second aspect of the technology proposed herein,
or obtained by the method according to the third aspect of the technology proposed herein.

Accordingly, the present invention is based on the discovery by the present inventors that production of SGA in the plant can be reduced or prevented by reducing or inhibiting the expression of at least one gene selected from *SMO1-L, DWF7-L,* and *TAM iso 2.*

As seen in the examples, inactivation of at least one of these genes reduces the content of SGA in the plant cell and in the plant, including in tubers, without significantly affecting plant growth including tuber yield. In contrast, the other genes tested in example 1, i.e. *DVVF1-L, 16DOX* and *CYP88B1,* provided less advantageous properties seen overall.

The genetically modified plant cell, as well as the genetically modified plant, is not exclusively obtained by means of an essentially biological process.

Genetically modified plant cell and genetically modified plant means that the genome of the plant cell and the plant has been purposefully genetically modified.

The reduced content of at least one steroidal glycoalkaloid is to be considered in comparison to a wild-type plant cell or wild-type plant or part of wild-type plant. A wild-type plant cell or wild-type plant corresponds to the plant cell prior to the modification of the genome and to a plant obtained from a plant cell that has not had its genome modified.

A plant cell may comprise or be comprised by a plant protoplast, a tissue culture, and plant cells that are intact in plants.

The term plant covers all parts of the plant including a flower, a seed, a leaf, a stem, a root, and a tuber. A part of the plant may further comprise a plant callus or a plant clump.

Accordingly, the reduced content of at least one steroidal glycoalkaloid may apply to the whole of the plant, or to a part of the plant. A part of the plant may include a flower, a seed, a leaf, a stem, a root, and a tuber. Preferably the part of the plant is a leaf or a tuber, more preferably a tuber.

Accordingly, the plant may have a reduced content of at least one steroidal glycoalkaloid by having a reduced content of at least one steroidal glycoalkaloid in a tuber, or the tubers, of the plant.

Alternatively, or additionally, the plant may have a reduced content of at least one steroidal glycoalkaloid by having a reduced content of at least one steroidal glycoalkaloid in a leaf, or the leaves, of the plant.

Alternatively, or additionally, the plant may have a reduced content of at least one steroidal glycoalkaloid by having a reduced content of at least one steroidal glycoalkaloid in a flower or flowers, a seed or seeds, a stem or stems, or a root or roots, of the plant.

A wild-type plant, in particular a wild-type *Solanum tuberosum* plant, i.e. a potato plant, may have a total content of steroidal glycoalkaloids in the leaves of 1500 to 1600 mg/kg, such as 1525 to 1575 mg/kg, such as 1565 mg/kg leaf.

Such a wild-type plant may also have a total content of steroidal glycoalkaloids in the tubers of 80-100 mg/kg, such as 85-95 or 90.7 mg/kg tuber.

Generally, the total content of steroidal glycoalkaloids in the plant cell and in the plant or part of the plant is below 1500 mg/kg, preferably below 1000 mg/kg, more preferably below 500 mg/kg, most preferably below 250 mg/kg of the plant cell, plant or part of the plant.

Alternatively, the total content of the steroidal glycoalkaloids α-solanine and α-chaconine in the plant cell and in the plant or part of the plant is below 1500 mg/kg, preferably below 1000 mg/kg, more preferably below 500 mg/kg, most preferably below 250 mg/kg of the plant cell, plant or part of the plant.

Further alternatively, the content of the steroidal glycoalkaloid α-solanine in the plant cell and in the plant or part of the plant is below 600 mg/kg, preferably below 200 mg/kg, more preferably below 125 mg/kg, most preferably below 80 mg/kg of the plant cell, plant or part of the plant.

Further alternatively, the content of the steroidal glycoalkaloid α-chaconine in the plant cell and in the plant or part of the plant is below 1000 mg/kg, preferably below 300 mg/kg, more preferably below 200 mg/kg, most preferably below 150 mg/kg of the plant cell, plant or part of the plant.

The above ranges in particular relate to the content (total, α-solanine and α-chaconine α-solanine alone and α-chaconine alone) in the leaves of the plant, more particularly to in the leaves of a genetically modified *Solanum tuberosum* plant, i.e. a genetically modified potato plant according to the fourth aspect of the technology proposed herein.

For a tuber or the tubers of a genetically modified plant, the total content of steroidal glycoalkaloids is preferably below 90 mg/kg, preferably below 45 mg/kg, more preferably below 25 mg/kg, even more preferably below 15 mg/kg, most preferably below 10 mg/kg tuber or tubers.

These ranges in particular relate to a tuber or the tubers of a genetically modified *Solanum tuberosum* plant, i.e. a genetically modified potato plant according to the fourth aspect of the technology proposed herein.

The plant cell may be provided from a wild-type plant. Sources for obtaining the plant cell include plant protoplasts, plant cells of tissue culture from which plants can be regenerated, plant calli, plant clumps and plant cells that are intact in plants or parts of plants such as pollen, meristems, flowers, seeds, leaves, stems, and the like.

The method according to the first aspect of the technology proposed herein can be formed on a single plant cell or on a plurality of plant cells. The method may also be applied to one or more plant cells present in a plant.

Modifying the genome of the plant cell comprises making changes in the genes or part of genes in the genome of the plant cell. This may for example include modifying a part of the gene such as a regulatory element or sequence.

The genome of the plant cell is preferably stably modified such that the modifications are inherited if the plant cell is propagated.

The at least one gene is selected from the group consisting of *SMO1-L, DWF7-L,* and *TAM iso 2.* As will be evident from the below, the gene DWF7-L comprises the genes *DVVF7-L (a),* and *DWF7-L (b).*

The genes all encode proteins acting in the pathway of the sterol and SGA biosynthesis. The genes are listed below with the corresponding encoded enzymes:
*SMO1-L* (sterol methyl-oxidase 1-like, SMO1-1-like),
(GENE ID: PGSC0003DMG400012763).

It is hypothesized that SMO1-L uses 24, 25-dihydrolansterol as substrate to produce 31-nor dihydroanosterol in the pre-cholesterol pathway. It is further hypothesized that SMO1-L uses cycloartenol as substrate to produce 31-nor cycloartenol in the pre-cholesterol pathway.

*DVVF7-L* (Δ7-C5 sterol desaturase-like) comprises two variants:
*DVVF7-L (a)* (Δ7-C5 sterol desaturase-like) (GENE ID: PGSC0003DMG400026401), and
*DWF7-L (b)* (*Δ7*-*C5 sterol desaturase-like)* (GENE ID: PGSC0003DMG402026400)

It is hypothesized that *DWF7-L* (*DWF7-L* (a) and *DWF7-L (b))* use lathosterol as substrate to produce 7-dehydrocholesterol in the pre-cholesterol pathway.
*TAM iso 2* (GABA transaminase isoform2, GAME12/PGA4),
(GENE ID: PGSC0003DMG400025228)

It is hypothesized that *TAM iso* 2 uses 16,22-dihydroxy-26-oxy-cholesterol as substrate to produce 16,22-dihydroxy-26-amino-cholesterol in the post-cholesterol pathway.

The reduced or inhibited expression of the at least one gene may comprise a decreased incidence or success in transcription, a decreased or absent enzymatic activity of the protein encoded by the at least one gene, or an absence of part or whole of the gene sequence.

The reduced or inhibited expression of the at least one gene may for example be obtained by gene silencing, antisense, RNA interference (RNAi), virus-induced gene silencing (VIGS), small RNA-mediated post-transcriptional gene silencing, transcription activatorlike effector nuclease (TALEN) gene editing techniques, clustered Regularly Interspaced Short Palindromic Repeat (CRISPR/Cas) gene editing techniques, and/or zinc-finger nuclease (ZFN) gene editing techniques.

Accordingly, the genome of the plant cell may be modified such that the reduced or inhibited expression is obtained at the gene level, e.g., by removing or altering the gene so as to affect the abundance and function of the protein produced from the at least one gene. Additionally, or alternatively, the reduced or inhibited expression may be provided in the transcription stage, e.g., by modifying the gene so as to decrease the probability that the at least one gene is transcribed to form mRNA.

Additionally, the decreased or inactivated expression may be provided at the mRNA stage by decreasing the likelihood that mRNA is ever translated into the protein, e.g., translational control, or by causing the mRNA to degrade fast.

The genetically modified plant cell may be cultivated or regenerated to obtain the genetically modified plant.

Preferably the at least one steroidal glycoalkaloid is selected from the group consisting of α-solanine, α-chaconine, solmargine, solasonine, tomatine and tomatidine.

More preferably the at least one steroidal glycoalkaloid comprises one or both of α-solanine and α-chaconine as these are the main steroidal glycoalkaloid in *Solanum tuberosum* plants. Further, as shown in example 1, the content of these glycoalkaloids may in particular be reduced.

Preferably the gene is selected from the group consisting of *SMO1-L* and *DVVF7-L,* more preferably the gene is *SMO1-L.* These genes, and in particular *SMO1-L,* have the best results as regards concentration of steroidal glycoalkaloid and growth, e.g. plant height and tuber yield.

Preferably step ii comprises mutating or excising at least a part of the gene.

Accordingly, in the genetically modified plant cell the gene has preferably been mutated or at least a part of the gene has been excised.

In the genetically modified plant cell, the gene has preferably been mutated in vitro. In other words, in the genetically modified cell, the gene has not been mutated spontaneously.

When the gene has been mutated, the gene is preferably selected from the group consisting of *SMO1-L* and *DVVF7-L.*

Preferably step ii is performed using CRISPR/Cas.

Accordingly, in the genetically modified plant cell, the genome of the plant has preferably been modified using CRISPR/Cas.

This is advantageous as it allows the excision of the at least one gene which provides a complete inactivation of this gene.

The CRISP/Cas may preferably be CRISPR/Cas9 or CRISPR/Cas12, such as CRISPR/Cas12a or CRISPR/Cas12b.

The genetically modified plant cell may be from the family *Solanaceae,* preferably from the genus *Solanum,* more preferably from the species *Solanum tuberosum.* In other words, the genetically modified plant cell may be a genetically modified potato cell.

Accordingly, the genetically modified plant may be from the family *Solanaceae,* preferably from the genus *Solanum,* more preferably from the species *Solanum tuberosum.* In other words, the genetically modified plant may be a genetically modified potato plant.

A fifth aspect of the technology proposed herein relates to a use of a genetically modified plant according to the fourth aspect of the technology proposed herein or part thereof, preferably a tuber thereof, in a process for producing starch, wherein preferably at least one of amino acids, minerals, proteins, and dietary fibers is obtained and collected as a by-product of the process.

This is advantageous in that the reduced content of steroidal glycoalkaloids in the plant makes it easier to obtain and collect by-products of the process, in particular amino acids, minerals, proteins, and dietary fibers.

The use according to the fifth aspect may alternatively be considered a method of using a genetically modified plant according to the fourth aspect of the technology proposed herein, preferably a part thereof, more preferably a tuber thereof, in a process for producing starch, wherein preferably the method comprises that at least one of amino acids, minerals, proteins, and dietary fibers is obtained and collected as a by-product of the process.

The part of the plant is preferably a starch containing part, such as the part of the plant that has the highest content of starch.

Preferably a tuber of the plant is used as tubers generally have a high starch content. The process for producing starch may comprise on or more of the steps of:
- cleaning of the plant, part thereof or tuber thereof,
- disrupting the cell walls (rasping) of the plant, part thereof or tuber thereof, to produce a juice and a pulp,
- optionally separating juice and pulp,
- starch extraction by washing the pulp to produce a starch milk,
- starch milk raffination such as by removing fibers from the starch milk, and
- dewatering of refined starch milk and starch drying.

At least one of amino acids, minerals, proteins, and dietary fibers is obtained and collected as by-product of the process. The at least one of amino acids, minerals, proteins, and dietary fibers may be obtained and collected from an aqueous solution or suspension, in particular the juice, obtained when processing the plant, part of plant, or tuber of plant for extracting the starch.

Preferably at least one of proteins, and dietary fibers are obtained and collected.

A sixth aspect of the technology proposed herein concerns the use of a genetically modified plant according to the fourth aspect of the technology proposed herein, preferably a part thereof, more preferably a tuber thereof, in a process for producing a food product, wherein the food product preferably is selected from the group consisting of peeled potato tubers, mashed potato or instant mashed potatoes, deep-fried potato products such as pommes frites or crisps, and potato flakes.

This is advantageous in that the reduced content of steroidal glycoalkaloids in the plant makes provides for a safer food product.

The use according to the sixth aspect may alternatively be considered a method of using a genetically modified plant according to the fourth aspect of the technology proposed herein, preferably a part thereof, more preferably a tuber thereof, in a process for producing a food product, wherein the food product preferably is selected from the group consisting of peeled potato tubers, mashed potato or instant mashed potatoes, deep-fried potato products such as pommes frites or crisps, and potato flakes.

A seventh aspect, corresponding to the first and aspect, of the technology proposed herein concerns the use of the reduction or inhibition of the expression of at least one gene selected from the group consisting of *SMO1-L, DWF7-L,* and *TAM iso 2* in the genome of a plant cell for reducing the content of at least one steroidal glycoalkaloid in the plant cell.

An eighth aspect of the technology proposed herein concerns progeny of a plant according to the fourth aspect of the technology proposed herein.

A ninth aspect of the technology proposed herein concerns a seed obtained from a plant according to the fourth aspect of the technology proposed herein.

A tenth aspect of the technology proposed herein concerns a cutting or graft of a plant according to the fourth aspect of the technology proposed herein.

An eleventh aspect of the technology proposed herein concerns a callus of a plant according to the fourth aspect of the technology proposed herein.

Alternative first and second aspects of the technology proposed herein concerns a method of obtaining a plant cell having reduced content of at least one steroidal glycoalkaloid, wherein the method comprises the steps of:
i. providing a plant cell, and
ii. providing, in the plant cell, at least one agent that:
   a. reduces or inhibits the enzymatic activity of a protein encoded by at least one gene selected from the group consisting of *SMO1-L, DWF7-L,* and *TAM iso 2,* or
   b. reduces or inhibits the expression at least one gene selected from the group consisting of *SMO1-L, DWF7-L,* and *TAM iso 2,*
      and
a plant cell having reduced content of at least one steroidal glycoalkaloid, wherein plant cell comprises at least one agent that:
a. reduces or inhibits the enzymatic activity of a protein encoded by at least one gene selected from the group consisting of *SMO1-L, DWF7-L,* and *TAM iso 2,* or
b. reduces or inhibits the expression at least one gene selected from the group consisting of *SMO1-L, DWF7-L,* and *TAM iso 2.*

The alternative first and second aspects of the technology proposed herein provide plant cells useable in the third and fourth aspects of the technology proposed herein as described further above.

### Brief description of the drawings and detailed description

A more complete understanding of the abovementioned and other features and advantages of the technology proposed herein will be apparent from the following detailed description of preferred embodiments in conjunction with the appended drawings, wherein:
Fig. 1A-1B show the structure of the targeted key genes positioned in the pre- (Fig. 1A) and post-cholesterol pathway (Fig. 1B) of SGA biosynthesis, with sgRNAs and analytic primers indicated with vertical and horizontal arrows, respectively.

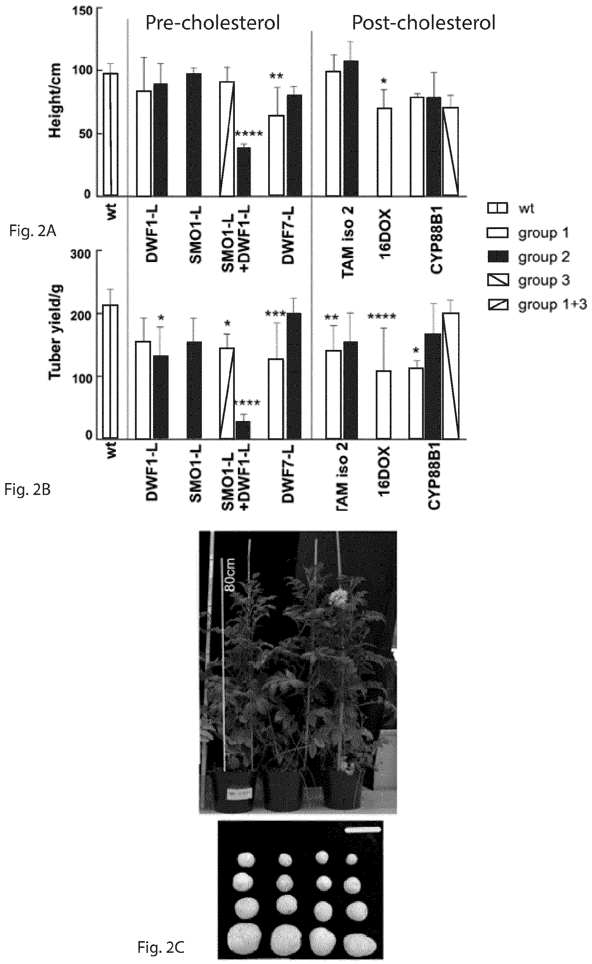

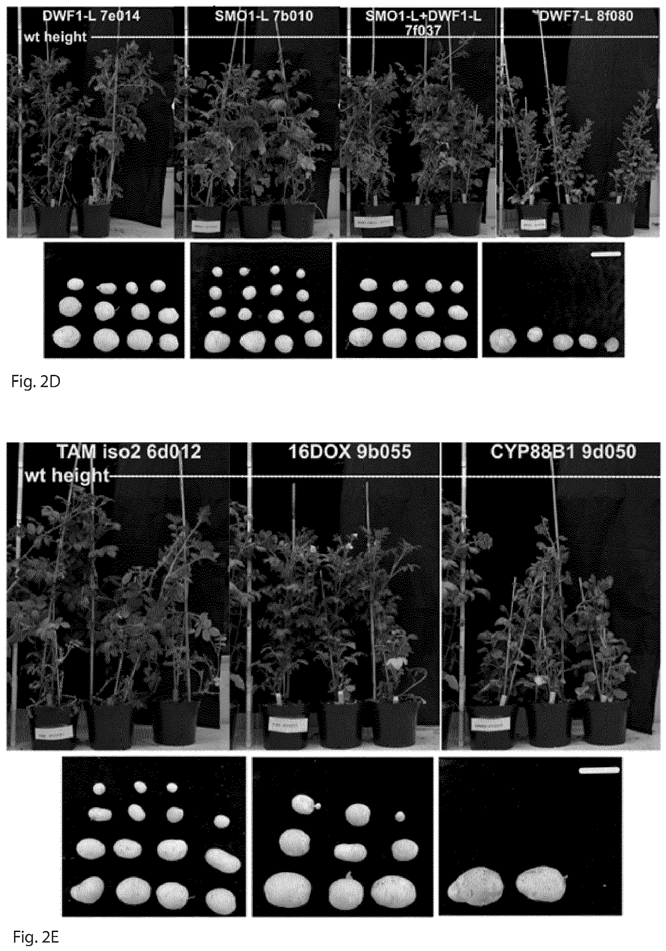

Fig. 2A-2E show mutated potato events generated by DNA-free CRISPR/Cas9 targeting key genes in the SGA pre- and post-cholesterol pathway, respectively. Fig. 2A: Height of four-month-old plants from the wild-type and selected 36 mutated events growing under controlled conditions. Fig. 2B: Tuber yield of wild-type and 36 mutated events harvested after six-month cultivation. Fig: 2C General appearance of wild-type plants (three-month-old) and tubers at harvest. Fig. 2D and 2E: General appearance of representative mutated events (three-month-old) and their corresponding tubers (at harvest) with different genes in the pre-cholesterol pathway (Fig. 2D) targeted and in the post- cholesterol pathway (Fig. 2E). Data in Fig. 2A and 2B represent the mean ± s.d, n=3-27 depending on the number of mutated events in each mutation group for each gene. P values were calculated using one-way ANOVA followed with Dunnett's test and indicated with asterisk (*P < 0.05, **P < 0.01, ***P < 0.001, ****P < 0.0001). Bars in tubers are 5 cm.
Fig 3A-3B shows detailed information regarding the height and tuber yield of all 36 individual events. One-way ANOVA followed with Dunnett's test was used for statistical analysis and P values are noted with asterisk (*P < 0.05, **P < 0.01, ***P < 0.001, ****P < 0.0001).
Fig. 4A shows TGA levels of individual mutated events. Statistical analysis was performed using one-way ANOVA compared to wild-type untreated tuber samples, and P values < 0.05 are noted with asterisk.
Fig. 4B shows the level of TGAs in the tubers at harvest (untreated), tubers subjected to wounding treatment for 2 days (wound) and light exposure for 8 days (light) for the genes *DWF7-L, SMO1-L and TAM iso 2.*

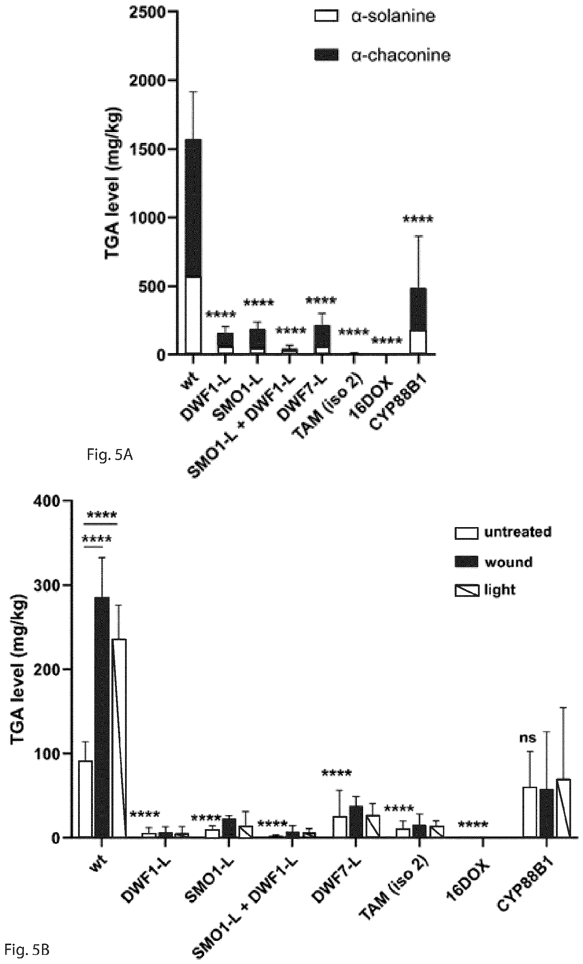

Fig. 5A-5B shows mean SGA level in selected 36 mutated events. Fig. 5A: The level of predominant SGAs (α-solanine and α -chaconine) and TGAs (total SGAs) in the leaves mutated potato events (8-week-old). Fig. 5B: The level of TGAs in the tubers at harvest (untreated), tubers subjected to wounding treatment for 2 days (wound) and light exposure for 8 days (light). Data in Fig. 5A and 5B represent the mean ±s.d, n=2-16 depending on the number of mutated events for each gene. Statistical analysis was performed using one-way ANOVA followed by Dunnett's post hoc test and P values are noted with asterisk (ns, not significant; *P < 0.05, **P < 0.01, ***P < 0.001, ****P < 0.0001).
Fig. 6 shows the sterol profile in the mutated events. Relative metabolite level of eight sterols in the leaves of mutated plants. Values represent mean ±s.d. of n=2-16 depending on the number of mutated events for each gene. One-way ANOVA followed with Dunnett's test was used for statistical analysis and P values are noted with asterisk (*P < 0.05, **P < 0.01, ***P < 0.001, ****P < 0.0001).

### EXAMPLE 1 - CRISPR/Cas9 -mediated generation of glycoalkaloid-free potato plants by mutation of key genes in the glycoalkaloid biosynthesis pathway

### 1.1 Materials and methods

### 1.1.1 Plant materials and growing conditions

*Solanum tuberosum* L. starch potato cultivar Kuras was used in this study. *In vitro* shoots were cultured in a controlled chamber under LD (16-h light/8-h dark) conditions with a photosynthetic photon flux of 80 µmol photons m⁻²s⁻¹ of light at 24 °C (dark at 18 °C). Greenhouse cultivation of mutants and wild-type plants in triplicates was under the same conditions as in vitro conditions, except for light supplied by natural daylight and supplemented with mercury lamps (ca. 70 µmol m⁻²s⁻¹). All mutated events were cultivated simultaneously and subjected to uniform growth conditions in the greenhouse.

### 1.1.2 Generation of CRISPR/Cas9 mutated plants

Genomic DNA was extracted from cv. Kuras leaf tissue using the GeneJet Plant Genomic DNA Purification Mini Kit (Thermo Fisher Scientific, Waltham USA) and used for amplification of the target regions in different genes to identify homologous regions between all four alleles. For each gene, two sgRNAs were designed targeting different exons in the allelic homologous regions using CRISPR RGEN Gas-Designer (http://www.rgenome.net/cas-designer/) and CRISPOR (http://crispor.tefor.net/crispor.py). Mutations were induced in cv. Kuras through Polyethylene Glycol (PEG)-mediated protoplast transfection of ribonucleoproteins (RNPs). In vitro propagation, protoplast isolation, and shoot regeneration were performed as described in Nicolia et al. (2015; 2021). Transfection of RNPs to freshly isolated protoplasts was conducted as described in Andersson et al. (2018) using 0.1 nmol synthetic sgRNA (Synthego, Redwood city, CA, USA) and 5 µg TrueCutTM Cas9 v.2 (Thermo Fisher, Waltham, USA) per target. The transfection into protoplasts was conducted with 25% PEG (PEG 4000, Sigma-Aldrich, Germany) for 3 min under room temperature. Only one regenerated shoot was selected from single calli and transferred to root regeneration. High Resolution Fragment Analysis (HRFA) was performed for initial screening of mutants in regenerated shoots, followed by Sanger sequencing (Eurofins Genomics, Ebersberg, Germany) as described in Andersson et al. (2017). A list of primers and sgRNAs used in this study are tables 1 and 2 below which in the last column also gives the SEQ ID NO: (NO) for the respective sequence.

**Table 1. Primers used**

| **Gene** | **Forward primer (5' to 3)** | **Reverse primer (5' to 3')** | **use** | **NO** |
|---|---|---|---|---|
| Primers | | | | |
| *DWF1-L* | CGTCCTCAAGTTTCCAAGAT GTCG** | CATCGTGTTCCATCTGGCG TTG | Analytic | 1,2 |
| *DWF1-L* | AAAGGCAGGGTGACACCGA A** | GCATTGCTCGTACAGACTG TTATC | Analytic | 3, 4 |
| *SMO1-L* | GCCTTTTGAGAGTATTGAA GAGGCT* | TCATAACCATTGGTAAAGG GACC | Analytic | 5, 6 |
| *SMO1-L* | GACAAGTTTGCCCCTGCCA TC* | GCCAAGAAAAGAAGCTAAG CCC | Analytic | 7, 8 |
| *DWF7-L* | TCCGAGTATACCGTCGGAG AT* | GTGATAAATGTAGAAGCAC CAGAGG | Analytic | 9, 10 |
| *DWF7-L* | CCGTGGTACTGTGCCCTT** | GCATGCAGATATTTGTACA GAGG | Analytic | 11, 12 |
| *TAM iso 2* | TTGCTTCATCTTTCTCTCTT GTTCT | CAAGGTACTTCTTTCCATTT ACGTC | Seq exon 2 | 13, 14 |
| *TAM iso 2* | AGAAGCATGGGATGTTGGT AAGT | TCAGTATCCTTCAATGCTTT CCCA | Seq. exon 2 | 15, 16 |
| *TAM iso 2* | CTAGTTAACAAAATATGTAC ACG* | GCATATAACTTAAAACTCGT AAC | Analytic | 17, 18 |
| *TAM iso 2* | AGAAGCATGGGATGTTGGT AAGT* | GCAGACTTTGTCCTTAACT GGT | Analytic | 19, 20 |
| *16DOX* | AACTGGTCAAGCACATTAG AAGC* | GTTCTTCACCATTAGCTTTT CCCA | Analytic | 21, 22 |
| *16DOX* | GGAGGTTATTGGTGCATAT GGAG** | CACCAATCCATTTGTCATC CTTC | Analytic | 23, 24 |
| *CYP88 B1* | TGGGAAGCCAACAATTATG GTG* | TGGCAAGTTGTGAACAGCA C | Analytic | 25, 26 |
| *CYP88 B1* | GCTCGAAAAGTACTAGGAG GG** | AGCAACAGGTTCAAAACCA GC | Analy tic | 27, 28 |

| | | | | |
|---|---|---|---|---|
| Forward primers with FAM (*) or HEX (**) tagged for HRFA. | | | | |

**Table 2. sgRNA used**

| **Gene** | **sgRNA** | **Sequence (5' to 3')** | **use** | **SEQ ID NO:** |
|---|---|---|---|---|
| *DWF1-L* | sg3 | CTTCCTCCTAGGGTGAACGG | targeting exon 2 | 29 |
| *DWF1-L* | sg4 | CATCAAATGGCTCACCCCTC | targeting exon 3 | 30 |
| *SMO1-L* | sg1 | CTAGGGAGAAATTTGACATT | targeting exon 1 | 31 |
| *SMO1-L* | sg2 | TTGCAGCACCTTATGCCCAC | targeting exon 2 | 32 |
| *DWF7-L* | sg11 | TGGCTCCGTAACTACATTGG | targeting exon 1 | 33 |
| *DWF7-L* | sg12 | TAGTGGAGTTTGGAATCTAC | targeting exon 2 | 34 |
| *TAM iso 2* | sg5 | TGCGGCATGGATGATTGATA | targeting exon 2 | 35 |
| *TAM iso 2* | sg6 | TCAAGACTCAAGGTGAATGG | targeting exon 16 | 36 |
| *16DOX* | sg7 | CCGATGGTCTTTCATGCACT | targeting exon 2 | 37 |
| *16DOX* | sg8 | AGGGTGAGCAAAGTTATCTT | targeting exon 4 | 38 |
| *CYP8881* | sg9 | GATGAAGAATATCTTGAAAG | targeting exon 2 | 39 |
| *CYP8881* | sg10 | GGATGGAAAATCAATGCCAA | targeting exon 3 | 40 |

### 1.1.3 Glycoalkaloid analyses

Thin-layer chromatographic (TLC) analysis was employed for rapid screening of SGA levels in 5-week-old in vitro mutated events, with α-chaconine and α-solanine (Sigma) as internal standards, according to Simonovska & Vovk (2000) with adaptations for in vitro plant materials.

Leaf and tuber samples were collected from greenhouse-grown plants at 8 weeks and 6 months, respectively. Treated tuber samples were prepared subjected to SGA-inducing conditions, wounding and light exposure, respectively, as described in (Petersson et al. 2013b). All the samples after preparation were immediately frozen in liquid nitrogen and stored at -70 °C for subsequent analyses, including SGA quantification, sterol analyses and metabolome analyses.

The determination of SGAs (α-chaconine and α-solanine) was conducted by LC-MS analysis in leaf and tubers samples of both mutated events and wild-type plants essentially as described in Merino et al. (2023).

### 1.1.4 Sterol analyses

The determination of sterols was conducted by GC-MS analysis essentially as described in Merino et al. (2023).

### 1.2 Results and discussion

### 1.2.1 Generation of potato events with induced mutations in key genes of the SGA biosynthesis via DNA-free CRISPR/Cas9

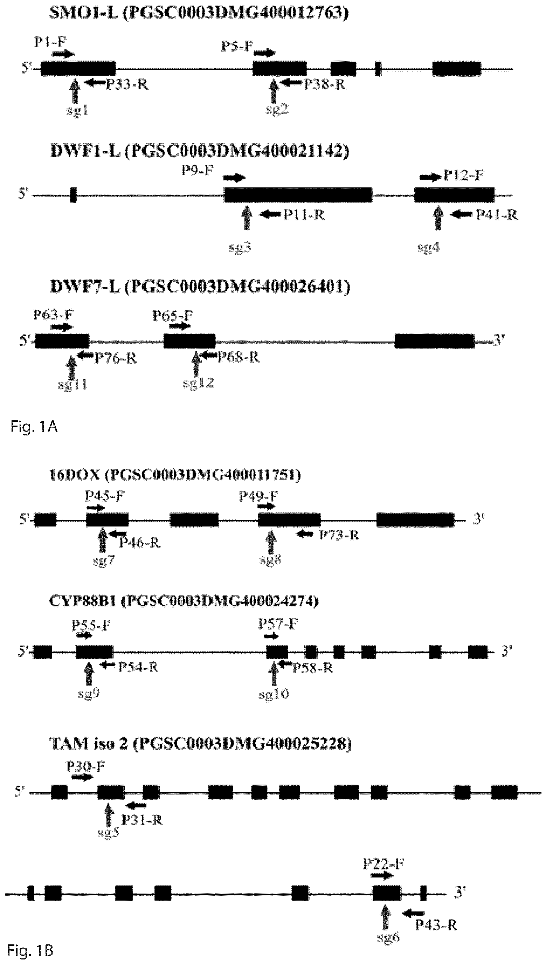

To generate SGA-free potato mutants, six genes encoding enzymes with a key role in either the pre- or post-cholesterol part of the SGA biosynthesis pathway were targeted by CRISPR/Cas9-mediated mutagenesis. The genes targeted, acting in the pre-cholesterol pathway were *DWF1-L, SMO1-L* and *DWF7-L*; and *TAM isoform 2 (GAME12), 16DOX (GAME11),* and *CYP8881* were acting in the post-cholesterol pathway of SGA biosynthesis. For each gene, two different sgRNAs were designed, each of which targeted different exons, and were used as single targets to knock out the corresponding key gene (Fig. 1). In addition, a double gene knockout strategy was undertaken for *SMO1-L* + *DWF1-L,* in this case by multiplexing two sgRNAs in different combinations. HRFA was employed for initial screening of mutations in the regenerated shoots, and the editing efficacy was calculated based on the number of shoots where at least one allele was mutated. The types of mutation were divided into three groups. Group 1, full knockouts where the indels were out of frame in all alleles. Group 2, full knockouts where at least one allele was an in-frame indels. Group 3, partially mutated with at least one wild-type allele unedited.

The results for the editing efficacy of each sgRNA targeting these six genes are shown in table 3. The highest editing efficiency was found in TAM (iso 2) regenerated shoots, followed by *DVVF7-L* and *DWF1-L,* with efficiencies of 49.5%, 48.5%, and 37.1%, respectively. For *SMO1-L* and *CYP8881* regenerated shoots, the editing efficiency was only 20.4% and 19.9%, respectively. For *SMO1-L,* in particular, mainly partially mutated events with two or three wild-type alleles remaining (group 3) were obtained. Given the limited efficacy of both sgRNAs in *SMO1-L,* only 57 out of 328 events (17.4%) exhibited double-mutants in both *SMO1-L* + *DWF1-L,* none of those events carried out-of-frame mutations in all alleles (group 1) for both genes.

**Table 3. The editing efficiency on regenerated shoots targeting different genes**

| **Target gene** | **sgRNA** | **Analyzed shoots** | **Edited shoots** | | **Editing efficiency** |
|---|---|---|---|---|---|
| *DWF1-L* | sg3 | 68 | 35 (51.47%) | | 37.06% |
| *DWF1-L* | sg4 | 75 | 18 (24.00%) | | |
| *SMO 1-L* | sg1 | 90 | 20 (22.22%) | | 20.42% |
| *SMO 1-L* | sg2 | 101 | 19 (18.81%) | | |
| *DWF7-L* | sg11 | 40 | 14 (35.00%) | | 48.51% |
| *DWF7-L* | sg12 | 94 | 51 (54.26%) | | |
| *TAM iso 2* | sg5 | 124 | 64 (51.61 %) | | 49.45% |
| *TAM iso 2* | sg6 | 58 | 26 (44.83%) | | |
| *16DOX* | sg7 | 67 | 7 (10.45%) | | 32.63% |
| *16DOX* | sg8 | 123 | 55 (44.72%) | | |
| *CYP8881* | sg9 | 115 | 26 (22.61%) | | 19.85% |
| *CYP8881* | sg10 | 152 | 27 (17.76%) | | |

| Double-mutant | | | Either gene | Both genes | |
|---|---|---|---|---|---|
| *SMO1-L* + *DWF1-L* | sg1 + sg3 | 70 | 21 (30.00%) | 5 (7.14%) | 41.16% either gene |
| *SMO1-L* + *DWF1-L* | sg1 + sg4 | 98 | 38 (38.78%) | 16 (16.33%) | |
| *SMO1-L* + *DWF1-L* | sg2 + sg3 | 60 | 27 (45.00%) | 11 (18.33%) | 17.38%, both genes |
| *SMO1-L* + *DWF1-L* | sg2 + sg4 | 100 | 49 (49.00%) | 25 (25.00%) | |

### 1.2.2 Initial screening of SGA levels of in vitro mutated events

A total of 96 events, targeted for the different genes in the pre- and post- cholesterol pathways, were selected based on mutation groups, with at least 2-3 events for each mutation group, thus encompassing mutations from full knockouts to partially mutated (mutations in the selected events are presented in table 4).

Initial screening of levels of α-solanine and α-chaconine was performed by TLC analysis of extracts from five-week-old in vitro rooted mutated events. The TLC results revealed a decrease in the SGA levels for 30 out of 50 events (60%) for the pre-cholesterol mutants, and 32 out of 46 (70%) events in the post-cholesterol mutants (table 4). There was a variation among mutated genes in the frequency of SGA reduction in the resulting events. For instance, the SGA levels were decreased in 100% of the selected events for both the *SMO1-L* + *DWF1-L* double-mutants and 16DOX, whereas only one event out of seven showed reduced SGA levels in the case of the *CYP8881* mutated events, the only full out-of-frame knockout obtained (table 4).

**Table 4. Mutations and TLC results on TGA levels of the selected 96 events**

| **Target gene** | **CRISPR event** | **Target site** | **sgRNA** | **Indels (bp)*** | **TGA level*** |
|---|---|---|---|---|---|
| Pre-cholesterol | | | | | |
| *DWF1-L* | 7c009 | exon2 | sg3 | -3;0 | + |
| *DWF1-L* | 7c010 | exon2 | sg3 | 0 | +++++ |
| *DWF1-L* | 7c016 | exon2 | sg3 | -4;0;1 | ++++ |
| *DWF1-L* | 7c019 | exon2 | sg3 | -8;-5;-3 | ++++ |
| *DWF1-L* | 7c024 | exon2 | sg3 | -3;-2;1 | +++++ |
| *DWF1-L* | 7c028 | exon2 | sg3 | -7;-5;-2;-1 | - |
| *DWF1-L* | 7c035 | exon2 | sg3 | -6;-2;-1 | +++ |
| *DWF1-L* | 7c039 | exon2 | sg3 | -3;-1;0;1 | +++++ |
| *DWF1-L* | 7c041 | exon2 | sg3 | -10;-5;-2;0 | +++++ |
| *DWF1-L* | 7c042 | exon2 | sg3 | -3 | ++++ |
| *DWF1-L* | 7c046 | exon2 | sg3 | -4;0 | +++ |
| *DWF1-L* | 7c051 | exon2 | sg3 | -4;-3;1 | +++ |
| *DWF1-L* | 7c055 | exon2 | sg3 | -3;-2;-1 | - |
| *DWF1-L* | 7c067 | exon2 | sg3 | -3 | +++ |
| *DWF1-L* | 7e014 | exon2 | sg3 | -2;-1;1 | - |
| *DWF1-L* | 7d010 | exon3 | sg4 | 0 | +++++ |
| *DWF1-L* | 7d017 | exon3 | sg4 | -2;-1 | ++ |
| *DWF1-L* | 7d024 | exon3 | sg4 | -3;-1 | + |
| *DWF1-L* | 7d025 | exon3 | sg4 | -5;-4;-1 | - |
| *DWF1-L* | 7d027 | exon3 | sg4 | -1;0 | +++++ |
| *DWF1-L* | 7d032 | exon3 | sg4 | -10;1;138 | - |
| *DWF1-L* | 7d037 | exon3 | sg4 | -5;-2;-1;0 | +++++ |
| *DWF1-L* | 7d041 | exon3 | sg4 | -7;-1;0 | +++ |
| *DWF1-L* | 7d069 | exon3 | sg4 | -5;-4; 0 | +++ |
| *DWF1-L* | 7f023 | exon3 | sg4 | -3;-2 | - |
| *DWF1-L* | 4h033 | exon3 | sg4 | -8;-2;1 | - |
| *SMO 1-L* | 7a078 | exon1 | sg1 | -6;-3;-1;0 | +++++ |
| *SMO 1-L* | 7b010 | exon2 | sg2 | -3;-1 | - |
| *SMO 1-L* | 7b044 | exon2 | sg2 | -4;-1;0;1 | + |
| *SMO1-L* + *DWF1-L* | 7f026 | exon1+3 | sg1 + sg4 | -3;-1;0 -6;-1 | - |
| *SMO1-L* + *DWF1-L* | 7f037 | exon1+3 | sg1 + sg4 | -20;-6;-3;0 -1 | - |
| *SMO1-L* + *DWF1-L* | 7f043 | exon1+3 | sg1 + sg4 | -7;-4;-1;0 -5;-4;-1 | - |
| *SMO1-L* + *DWF1-L* | 7g026 | exon2+2 | sg2 + sg3 | -4;-3 -10;-3 | - |
| *SMO1-L* + *DWF1-L* | 7h015 | exon2+3 | sg2 + sg4 | -5;-4;-1;0 -5;-3;-1;0 | - |
| *SMO1-L* + *DWF1-L* | 4h018 | exon2+3 | sg2 + sg4 | -6;-5;-3;1 -1;0 | - |
| *SMO1-L* + *DWF1-L* | 4h024 | exon2+3 | sg2 + sg4 | -4;-1;1 -5;0;1 | - |
| *SMO1-L* + *DWF1-L* | 4h037 | exon2+3 | sg2 + sg4 | -10;-5;-3;1 -5;-2;-1;0 | - |
| *SMO1-L* + *DWF1-L* | 4h043 | exon2+3 | sg2 + sg4 | -17;-3;-2;-1 -4;-3;-2 | - |
| *DWF7-L* | 9e003 | exon1 | sg11 | -4;-2;-1 | - |
| *DWF7-L* | 9e019 | exon1 | sg11 | -4;-1 | + |
| *DWF7-L* | 9e021 | exon1 | sg11 | -8;-2;0 | + |
| *DWF7-L* | 9e025 | exon1 | sg11 | -5;-2;-1 | +++++ |
| *DWF7-L* | 8f019 | exon2 | sg12 | -10;-8;0; | +++++ |
| *DWF7-L* | 8f025 | exon2 | sg12 | -5;-4;-3;1 | - |
| *DVVF7-L* | 8f034 | exon2 | sg12 | -4;-2;; | - |
| *DWF7-L* | 8f035 | exon2 | sg12 | -7;-2;-1;0 | ++++ |
| *DWF7-L* | 8f036 | exon2 | sg12 | -5;-2;-1 | - |
| *DWF7-L* | 8f038 | exon2 | sg12 | -5;-4;-3; | - |
| *DWF7-L* | 8f059 | exon2 | sg12 | -5;-3;-2; | - |
| *DWF7-L* | 8f080 | exon2 | sg12 | -5;-2;1; | - |

| Post-cholesterol | | | | | |
|---|---|---|---|---|---|
| *TAM iso 2* | 6a001 | exon2 | sg5 | -5;-4;0; | +++++ |
| *TAM iso 2* | 6a004 | exon2 | sg5 | -6;-4;1; | - |
| *TAM iso 2* | 6a011 | exon2 | sg5 | 0;3;; | - |
| *TAM iso 2* | 6a017 | exon2 | sg5 | -5;-3;-2; | - |
| *TAM iso 2* | 6b001 | exon2 | sg5 | -7;-5;-4;1 | - |
| *TAM iso 2* | 6b004 | exon2 | sg5 | -5;-1;0; | +++++ |
| *TAM iso 2* | 6b014 | exon2 | sg5 | -5;-2;0; | +++++ |
| *TAM iso 2* | 6b015 | exon2 | sg5 | -7;-5;-2;105 | - |
| *TAM iso 2* | 6b026 | exon2 | sg5 | -5;-2;0;1 | - |
| *TAM iso 2* | 6b027 | exon2 | sg5 | -4;-2;-1;0 | +++++ |
| *TAM iso 2* | 6b057 | exon2 | sg5 | -3;-2;; | - |
| *TAM iso 2* | 6b075 | exon2 | sg5 | -5;-4;-2; | - |
| *TAM iso 2* | 6b083 | exon2 | sg5 | -5;-4;0; | +++++ |
| *TAM iso 2* | 6b093 | exon2 | sg5 | -4;-3;; | ++ |
| *TAM iso 2* | 6d001 | exon16 | sg6 | -1;0;1; | +++++ |
| *TAM iso 2* | 6d003 | exon16 | sg6 | -4;1;12 | - |
| *TAM iso 2* | 6d006 | exon16 | sg6 | -3;-1;1; | - |
| *TAM iso 2* | 6d007 | exon16 | sg6 | -5;-2;-1;0 | +++++ |
| *TAM iso 2* | 6d010 | exon16 | sg6 | -13;-1;; | - |
| *TAM iso 2* | 6d012 | exon16 | sg6 | -3;-1;1; | - |
| *TAM iso 2* | 6d016 | exon16 | sg6 | -4;-1;; | + |
| *TAM iso 2* | 6d018 | exon16 | sg6 | -10;-3;-1; | - |
| *TAM iso 2* | 6d022 | exon16 | sg6 | -4;-3;-1;0 | +++++ |
| *TAM iso 2* | 6d031 | exon16 | sg6 | -5;-4;1; | - |
| *TAM iso 2* | 6d041 | exon16 | sg6 | -2;-1;1 | - |
| *TAM iso 2* | 6d042 | exon16 | sg6 | -4;-1;0;1 | - |
| *16DOX* | 8b015 | exon4 | sg8 | 3;-1;0 | - |
| *16DOX* | 8b026 | exon4 | sg8 | -20;-5;-2; | - |
| *16DOX* | 9b006 | exon4 | sg8 | -6;-2;0;1 | - |
| *16DOX* | 9b008 | exon4 | sg8 | -30;-29;-2;-1 | - |
| *16DOX* | 9b009 | exon4 | sg8 | -6;-2;-1 | - |
| *16DOX* | 9b014 | exon4 | sg8 | -2;-1;1; | - |
| *16DOX* | 9b022 | exon4 | sg8 | -7;-3;-2;-1 | - |
| *16DOX* | 9b023 | exon4 | sg8 | -8;-7;-4;-2 | - |
| *16DOX* | 9b029 | exon4 | sg8 | -6;-3;-2; | - |
| *16DOX* | 9b030 | exon4 | sg8 | -15;-7;-2; | - |
| *16DOX* | 9b040 | exon4 | sg8 | -10;-3;-2;0 | - |
| *16DOX* | 9b046 | exon4 | sg8 | -4;-1;; | - |
| *16DOX* | 9b055 | exon4 | sg8 | -2;-1;; | - |
| *CYP8881* | 9c006 | exon2 | sg9 | -6;-4;-3;-2;0 | ++++ |
| *CYP8881* | 9c068 | exon2 | sg9 | -4;-3;0;1 | +++++ |
| *CYP8881* | 8d025 | exon3 | sg10 | -2;0;1 | +++++ |
| *CYP8881* | 9d005 | exon3 | sg10 | -4;-3;-2; | +++ |
| *CYP8881* | 9d040 | exon3 | sg10 | -9;-4;-1; | +++++ |
| *CYP8881* | 9d050 | exon3 | sg10 | -4;-2;; | - |
| *CYP8881* | 9d054 | exon3 | sg10 | -4;-1;0; | +++++ |

| | | | | | |
|---|---|---|---|---|---|
| * The indels in the mutated events were indicated by positive values as insertions and negative values as deletions, "0" represents the unedited wild-type allele/alleles. In the events lacking "0", no wild-type allele is present. Note that two or more events might have the same size of indels. **TGA levels estimated by TLC analysis, from highest level defined as "+++++" (e.g. TGA levels in wild-type plants) to lowest "-" (no TGAs detected or below the detection level of TLC analysis). | | | | | |

### 1.2.3 Phenotypical variation among mutated potato events

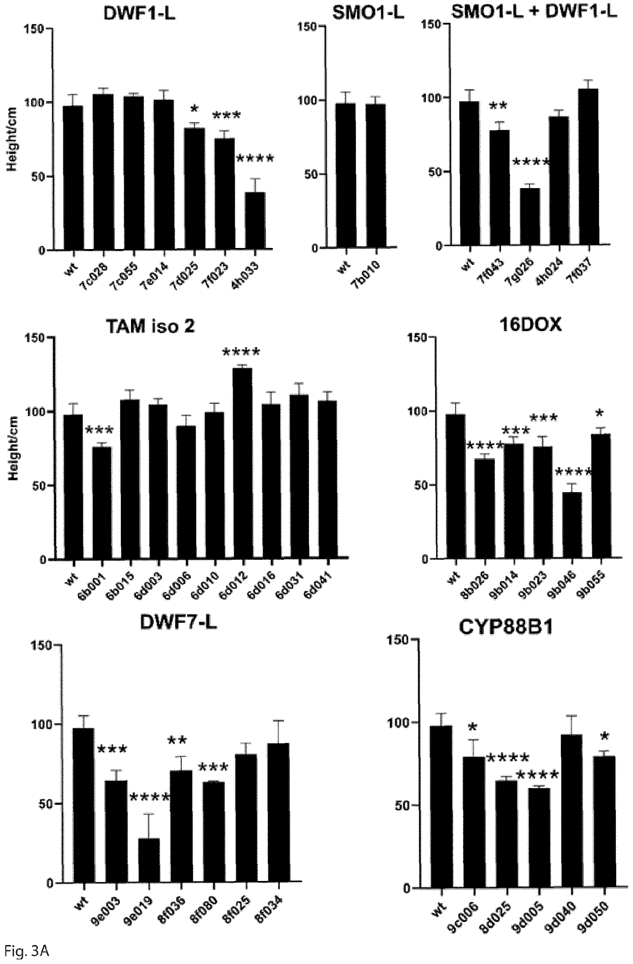

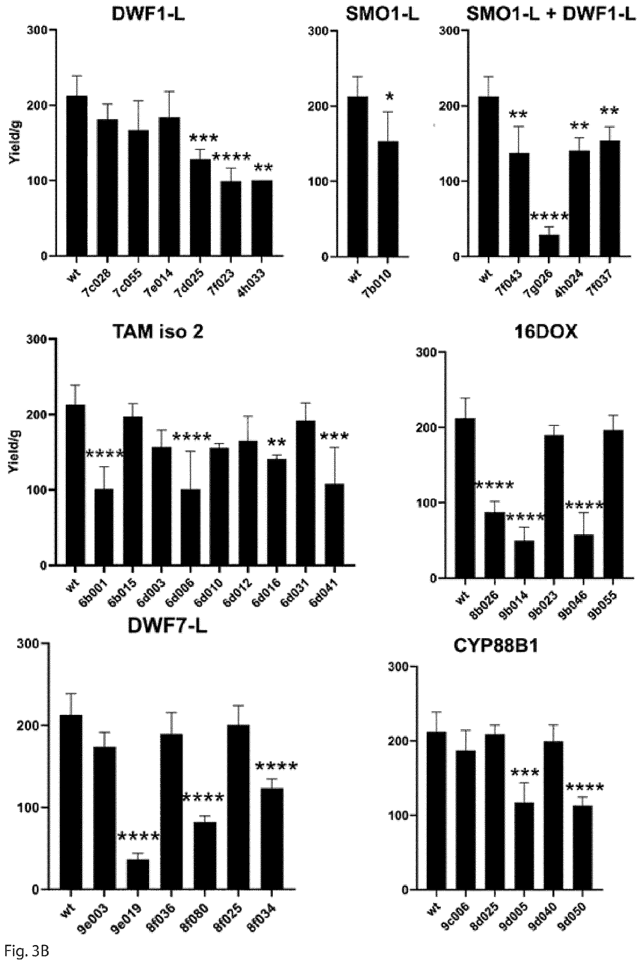

Based on low TGA levels by TLC screening (mainly events of mutation group 1 and 2), we subsequently selected 36 events listed in Table 5 below and monitored the plant growth and tuber yield under greenhouse conditions. The plant height of the mutated events was in most cases, not different from that of the wild-type plants (Fig. 2A, Fig. 3A). Exceptions were full knockouts from *SMO1-L* + *DWF1-L* double-mutants (group 2), DWF7-L (group 1) and *16DOX* (group 1), which all were significantly 30-40% shorter than the wild-type plants. The tuber yield was more clearly impacted, particularly the events in the mutation group 1, with reductions ranging from 27% to 87% (Fig. 2B, Supp. Fig. 3B). The highest reduction in tuber yield was observed for double-mutants of *SMO1-L* + *DWF1-L,* where both genes were fully mutated (group 2).

Compared to the wild-type, the general appearance and phenotype of a majority of the events was normal, regardless if they were pre-cholesterol or post-cholesterol mutants (Fig. 2C-2E). Nonetheless, a few events displayed an abnormal phenotype, including a dwarfed growth pattern with small or irregular leaves. This was exemplified by event 4h033 from *DWF1-L* mutants, 9e019 and 8f080 (Fig. 2C) from *DVVF7-L* mutants, 7g026 from double-mutants of *SMO1-L* + *DWF1-L,* and 9b046 from *16DOX* mutants. Notably, among the tubers from mutated events, no unusual phenotypes were observed, except a few events such as 6d003 (*TAM iso* 2 mutant) and 7g026 (double-mutant of *SMO1-L* + *DWF1-L),* displaying slightly elongated tubers.

**Table 5. The selected 36 events targeting key genes in the SGA biosynthesis pathway of upstream and downstream of cholesterol via CRISPR/Cas 9 mutagenesis.**

| **Target gene** | **CRISPR event** | **Target site** | **sgRNA** | **Indels (bp)** | | **Mutation group** |
|---|---|---|---|---|---|---|
| Pre-cholesterol | | | | | | |
| *DWF1-L* | 7c028 | exon2 | sg3 | -7;-5;-2;-1 | | 1 |
| *DWF1-L* | 7c055 | exon2 | sg3 | -3;-2;-1 | | 2 |
| *DWF1-L* | 7e014 | exon2 | sg3 | -2;-1;1 | | 1 |
| *DWF1-L* | 7d025 | exon3 | sg4 | -5;-4;-1 | | 1 |
| *DWF1-L* | 7f023 | exon3 | sg4 | -3;-2 | | 2 |
| *DWF1-L* | 4h033 | exon3 | sg4 | -8;-2;1 | | 1 |
| *SMO 1-L* | 7b010 | exon2 | sg2 | -3;-1 | | 2 |
| *SMO1-L* + *DWF1-L* | 7f043 | exon1+3 | sg1 + sg4 | -7;-4; -1;0 | -5;-4; -1 | 3 + 1 |
| *SMO1-L* + *DWF1-L* | 7g026 | exon2+2 | sg2 + sg3 | -4;-3 | -10;-3 | 2 + 2 |
| *SMO1-L* + *DWF1-L* | 4h024 | exon2+3 | sg2 + sg4 | -4;-1;1 | -5;0;1 | 1 + 3 |
| *SMO1-L* + *DWF1-L* | 7f037 | exon1+3 | sg1 + sg4 | -20;-7; -3;0 | -1 | 3 + 1 |
| *DVVF7-L* | 9e003 | exon1 | sg11 | -4;-2;-1 | | 1 |
| *DVVF7-L* | 9e019 | exon1 | sg11 | -4;-1 | | 1 |
| *DVVF7-L* | 8f036 | exon2 | sg12 | -5;-2;-1 | | 1 |
| *DVVF7-L* | 8f080 | exon2 | sg12 | -5;-2;1 | | 1 |
| *DVVF7-L* | 8f025 | exon2 | sg12 | -5;-4;-3;1 | | 2 |
| *DVVF7-L* | 8f034 | exon2 | sg12 | -4;-2 | | 1 |

| Post-cholesterol | | | | | | |
|---|---|---|---|---|---|---|
| *TAM iso 2* | 6b001 | exon2 | sg5 | -7;-5;-4;1 | | 1 |
| *TAM iso 2* | 6b015 | exon2 | sg5 | -7;-5;2;105 | | 1 |
| *TAM iso 2* | 6d003 | exon16 | sg6 | -4;-2;1;12 | | 2 |
| *TAM iso 2* | 6d006 | exon16 | sg6 | -3;-1;1 | | 2 |
| *TAM iso 2* | 6d010 | exon16 | sg6 | -13;-1 | | 1 |
| *TAM iso 2* | 6d012 | exon16 | sg6 | -3;-1;1 | | 2 |
| *TAM iso 2* | 6d016 | exon16 | sg6 | -4;-1 | | 1 |
| *TAM iso 2* | 6d031 | exon16 | sg6 | -5;-4;1 | | 1 |
| *TAM iso 2* | 6d041 | exon16 | sg6 | -2;-1;1 | | 1 |
| *16DOX* | 8b026 | exon4 | sg8 | -20;-5;-2 | | 1 |
| *16DOX* | 9b014 | exon4 | sg8 | -2;-1;1 | | 1 |
| *16DOX* | 9b023 | exon4 | sg8 | -8;-7;-4;-2 | | 1 |
| *16DOX* | 9b046 | exon4 | sg8 | -4;-1 | | 1 |
| *16DOX* | 9b055 | exon4 | sg8 | -2;-1 | | 1 |
| *CYP8881* | 9c006 | exon2 | sg9 | -6;-4;-3;-2;0 | | 3 |
| *CYP8881* | 8d025 | exon3 | sg10 | -2;0;1 | | 3 |
| *CYP8881* | 9d005 | exon3 | sg10 | -4;-3;-2 | | 2 |
| *CYP8881* | 9d040 | exon3 | sg10 | -9;-4;-1 | | 2 |
| *CYP8881* | 9d050 | exon3 | sg10 | -4;-2 | | 1 |

The indels in the mutated events were indicated by positive values as insertions and negative values as deletions, "0" represents the unedited wild-type allele/alleles. In the events lacking "0", no wild-type allele is present. Note that two or more events might have the same size of indels.

### 1.2.4 Decreased SGA levels in pre- and post-cholesterol mutated events

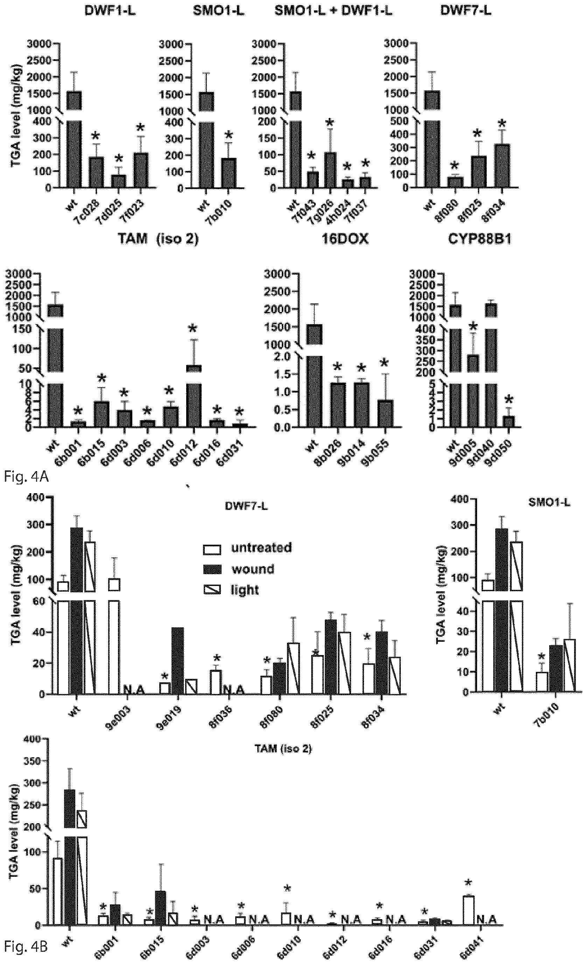

To further analyze the effect of mutations on SGA metabolism, the total SGA (TGA) levels were quantified using LC-MS analysis in both leaves and tubers of the 36 selected mutated events cultivated in greenhouse. In the leaf samples, a significant reduction of the TGA levels, down to less than 10% of the wild-type level, was observed in all 36 events. The most dramatic decreases were observed in the *16DOX* mutants, which displayed an almost complete absence of TGA, followed by mutants in the *TAM iso* 2 and double-mutants of *SMO1-L* + *DWF1-L* (Fig. 5A, Fig. 4A). The leaf LC-MS results of TGA levels were consistent with the initial TLC screenings. In the tuber samples, TGA levels were likewise extensively reduced in most of the events, except for *CYP8881* mutants, where the TGA levels remained similar to wild-type with the exception of one event (9d050) that showed no TGA (Fig. 4B). A total of 16 events were identified with a remarkable TGA decrease to less than 4 mg/kg FW in the tuber samples. These 16 events comprised five events from *DWF1-L* mutants (7c028, 7e014, 7d025, 7f023, and 4h033) (not shown in Fig. 4B), three from double-mutants of *SMO1-L* + *DWF1-L* (7f043, 4h024, and 7f037) (not shown in Fig. 4B), two from *TAM iso* 2 mutants (6d012 and 6d031), see Fig. 4B, all five events in *16DOX* mutants (8b026, 9b014, 9b023, 9b046, and 9b055) (not shown in Fig. 4B), and one knockout from *CYP8881* mutants (9d050) (not shown in Fig. 4B). All five events from *16DOX* displayed a near absence of TGA in both leaves and tubers and were all from mutation group 1.

To investigate the effects on SGA levels under SGA-inducing conditions, tubers from selected mutated events were subjected to, respectively, wounding and light exposure, indicated as "wound" and "light" in Fig. 5B (the results of individual events from *DVVF7-L, SMO1-L,* and *TAM iso* 2 are shown in Fig. 4B). The wild-type tubers displayed a statistically significant ca. 3-fold accumulation of SGAs under both wounding and light exposure treatments. By contrast, mutated events displayed no, or only a slight increase of the TGA level in the treated tubers, and the final TGA levels were in all cases statistically not different from those of the corresponding untreated controls.

### 1.2.5 Altered sterol profiles of pre- and post-cholesterol mutated events

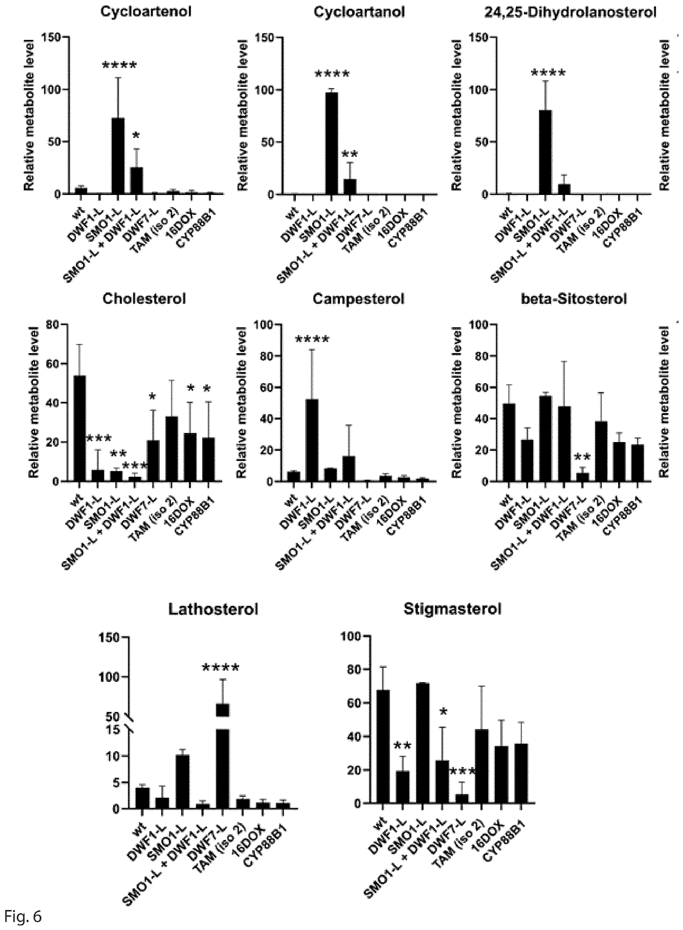

Sterol profiling was conducted on leaves from the mutated potato events to investigate the impact of the mutations in the key genes in sterol biosynthesis. The sterol profiles were altered in most of the events, particularly in the pre-cholesterol mutants (Fig. 6). Both cycloartenol and cycloartanol, the early intermediates of cholesterol, displayed a significant accumulation in the *SMO1-L* event, with levels 174 times and 12 times higher than those in the wild-type plants, respectively. A similar trend was observed in the double-mutants of *SMO1-L* + *DWF1-L* events, albeit with less accumulation of cycloartenol and cycloartanol. A significant increase in 24, 25-dihydrolanosterol was found in *SMO1-L* and double-mutants of *SMO1-L* + *DWF1-L* events, with levels 154 times and 18 times higher than that of wild-type, respectively, indicating that 24, 25-dihydrolanosterol is also the substrate of *SMO1-L.*

The reduction of cholesterol was stronger in the pre-cholesterol mutants compared to the post-cholesterol ones, with 17% and 54% of cholesterol maintained in the pre- and post-cholesterol mutants, respectively. This suggests that mutations in the genes in the pre-cholesterol pathway effectively blocked the synthesis of cholesterol. Notably, the only significant increase in lathosterol was displayed in the *DVVF7-L* events, providing support that *DVVF7-L* use lathosterol as substrate. Interestingly, with regard to the level of phytosterols, only the *DWF1-L* events exhibited a significant increase in campesterol, while the mutations in *DVVF7-L* resulted in a corresponding decrease in the sitosterol and the end-product stigmasterol.

### 1.2.6 Discussion

Here, we present targeted mutagenesis of six key genes involved in either the pre- or post-cholesterol pathway of SGA biosynthesis via CRIPSR/Cas9 DNA-free technology. TLC analyses of 96 mutated events revealed a considerable SGA variation both within and between different gene mutants. Nonetheless, the majority of gene mutants showed reduced SGA levels in both leaves and tubers, with several events displaying close to SGA-free tubers (SGA levels < 0.1 mg/kg FW) (Fig. 5A-5B, table 4). A correlation was identified between the number of mutated alleles (mutation group) and the degree of SGA reduction in the mutated events. Generally, mutations in all four alleles were necessary to achieve a significant reduction in SGA levels of the mutated events with different genes targeted, regardless of whether the mutations were out-of-frame (group 1) or included one in-frame induced mutation (group 2). Exceptions were *TAM iso* 2 and 16DOX mutated events, where group 3 mutations in some events also resulted in a significant decrease in SGA levels. Conversely, only the full out-of-frame (group 1) among CYP88B1 mutated events led to a significant reduction in SGA levels. Together this variability suggests that the genes encode proteins that limit the SGA biosynthesis to different degrees.

Among the 36 mutated events selected for more in-depth studies, the extent of SGA reduction was correlated with the position of the target genes in either the pre- or post-cholesterol pathway. Mutated events for post-cholesterol exhibited significantly lower SGA levels in both leaves and tubers, with tubers displaying a more pronounced reduction (Fig. 5A-5B). This might be because the genes in the post-cholesterol pathway are single copy and more specific to the SGA biosynthesis. Knocking out of these genes would result in a significant reduction in SGA levels. In contrast, some pre-cholesterol genes are duplicated, e.g. *DWF1-L* and *DWF1, SMO1-L* and *SMO1*, and *DWF7-L* and *DWF7,* each implicated in SGA and sterol biosynthesis, respectively. It can be speculated that when the chosen pre-cholesterol genes were knocked out, the duplicated counterparts involved in the sterol biosynthesis can to some extent compensate for the knocked-out genes involved in the SGA biosynthesis, thus maintaining a certain level of both sterols and SGAs in the mutants.

The SGA levels remained consistently low following wounding and light exposure treatments in all the mutated events (mainly with mutations group 1 or 2), compared the wild-type untreated tubers. Exceptions were noted in two *CYP8881* mutated events (9d050 and 9d040) with SGA levels comparable to wild-type in untreated tubers, where the response mirrored wild-type patterns, showing a significant increase after both wounding and light exposure. This suggests that the different key gene mutations have a capacity to block both the basal and the induced SGA synthesis.

The morphology of the majority of mutated plants and tubers remained similar to that of the wild-type, with some events displayed a slight reduction in height and/or decreased tuber yield (Fig. 2A-2E). However, no correlations were observed between reduced SGA content or even the absence of SGA and the plant height or tuber yield. Taken together, the distribution and low frequency of phenotypical variation among the mutants, suggest that the observed phenotypic effects were more related to random secondary effects from the mutant generation or regeneration processes that to their reduced SGA levels as such.

Both *SMO1-L* and *DVVF7-L* mutated events displayed a significant decrease in SGA levels, confirming their involvement in the SGA biosynthesis pathway. The accumulation of 24-25-dihydrolanosterol, cycloartenol, and cycloartanol in the *SMO1-L* mutated event indicated their roles as substrates for *SMO1-L* (Fig. 6). Likewise, the accumulation of lathosterol in the *DVVF7-L* mutated events confirmed the position of *DVVF7-L* in the cholesterol biosynthesis pathway (Fig. 6).

Double mutants for *SMO1-L* + *DWF1-L* displayed a higher degree of reduction compared to single mutants of either *SMO1-L or DWF1-L.* Together with the accumulation of cycloartenol and cycloartanol in both *SMO1-L* mutant and double mutants, our findings propose that both *SMO1-L* and *DWF1-L* contribute to the conversion of cycloartenol to 31-nor cycloartanol. The full-in-frame knockout in both *SMO1-L* and *DWF1-L,* event 7g026, was the only we obtained, resulting in severe dwarfism and significantly reduced tuber yield compared to the wild-type plants. This finding indicates the possibility that a full out-of-frame knockout in *SMO1-L* might be lethal, as indicated by the absence of such mutants. In summary, the double-knockout strategy for *SMO1-L* + *DWF1-L* appears to yield a more robust reduction in SGAs than the mutants targeting either *SMO1-L* or *DWF1-L.* However, this approach induced side effects on the phenotype of mutants, possibly due to the critical roles of duplicated genes in sterol biosynthesis.

In all nine *TAM iso 2* knockout events (mutation group 1 and 2) analyzed, the SGA levels were significantly reduced in both leaves and tubers, with a normal morphology of mutated plants and tuber. This was irrespective of whether the mutation position was in the early (exon 2) or later exon (exon 16) in the events. This indicated that TAM plays an important and indispensable role in the SGA biosynthesis.

*16DOX* was the first 2-oxoglutarate-dependent dioxygenase (2OGD) encoding gene identified with a role in the SGA biosynthesis pathway, and the *16DOX*-slienced potato and tomato plants displayed reduced SGA contents. The *16DOX* mutated events obtained in our study showed a significant reduction in SGA levels in leaves and the complete absence of SGAs in tubers. After greenhouse harvest, tubers from two 16DOX events were observed to exhibit suppressed sprouting and a prolonged sprouting time compared with the wild-type tubers (not shown).

The SGA level in the *CYP8881* (*GAME4*) out-of-frame knockout (event 9d050) was significantly decreased in both leaves and tubers. However, this event exhibited a dwarf phenotype and significant lower tuber yield (Fig. 2E). This observed phenotypic differences might be attributed to the accumulation of unknown compounds, particularly prominent in the full out-of-frame knockout compared to other mutated events.

In summary, the obtained SMO1-L, DWF7-L (a, b) and TAM iso 2-mutants, characterized by low SGA levels in the tubers, along with a normal phenotype and tuber yield, provide promising genetically modified plants for future industrial applications. This includes the development of entirely new food-grade protein and fiber products from industrial by-products and new food products with low SGA levels.

EXAMPLE 2 - Glycoalkaloid levels in wild-type potato (cv. Desiree) and RNAi or antisense transformants.

### 2.1 Materials and methods

In this example RNAi and antisense methods were used to obtain transformants which were compared to wild-type potato (cv. Desiree) as regards concentration of steroidal glycoalkaloids in leaf and tubers. Specifically, the gene TAM iso 2 was affected using antisense, and the gene SMO1-L was affected using RNAi.

### 2.1 Results

The following results were obtained:

**Table 6: HPLC glycoalkaloid levels (mg/kg) in leaf of wild-type vs antisense transformants - experiment A**

| **Sample** | | **mean** | **SD** | **% of wild-type** | **N (plants)** |
|---|---|---|---|---|---|
| Wild-type | | 465 | 27 | | 3 |
| asTAMiso2 | 2E | 147 | | 32% | 1 |
| asTAMiso2 | 10A | 248 | | 53% | 1 |
| asTAMiso2 | 2A | 300 | | 65% | 1 |
| asTAMiso2 | 2B | 357 | | 77% | 1 |

**Table 7: LC-MS glycoalkaloid levels (mg/kg) in leaf of wild-type vs RNAi transformants - experiment B**

| **Sample** | | **mean** | **SD** | **% of wild-type** | **N (plants)** |
|---|---|---|---|---|---|
| Wild-type | | 1598 | 204 | | 3 |
| iSMO1-L | #5 | 271 | | 17% | 1 |
| iSMO1-L | #4 | 268 | | 17% | 1 |
| iSMO1-L | #3 | 266 | | 17% | 1 |
| iSMO1-L | #21 | 433 | | 27% | 1 |
| iSMO1-L | #14 | 315 | | 20% | 1 |
| iSMO1-L | #9 | 496 | | 31% | 1 |

**Table 8: LC-MS glycoalkaloid levels (mg/kg) in leaf of wild-type vs RNAi transformants - experiment C**

| **Sample** | | **mean** | **SD** | **% of wild-type** | **N (plants)** |
|---|---|---|---|---|---|
| Wild-type | | 1737 | 925 | | 4 |
| iSMO1-L | #5 | 185 | | 11% | 1 |
| iSMO1-L | #4 | 213 | | 12% | 1 |
| iSMO1-L | #3 | 259 | | 15% | 1 |
| iSMO1-L | #21 | 271 | | 16% | 1 |
| iSMO1-L | #14 | 343 | | 20% | 1 |
| iSMO1-L | #9 | 380 | | 22% | 1 |

**Table 9: LC-MS glycoalkaloid levels (mg/kg) in tuber of wild-type vs RNAi transformants - experiment C**

| **Sample** | | **mean** | **SD** | **% of wild-type** | **N (plants)** |
|---|---|---|---|---|---|
| Wild-type | | 66 | 8 | | 3 |
| iSMO1-L | #5 | 20.6 | 0.4 | 31% | 2 |
| iSMO1-L | #4 | 12.8 | 1.4 | 19% | 2 |
| iSMO1-L | #3 | 11.6 | 2.5 | 18% | 2 |
| iSMO1-L | #21 | 18.6 | 2.6 | 28% | 2 |
| iSMO1-L | #14 | 7.6 | 0.6 | 12% | 2 |
| iSMO1-L | #9 | 8.4 | 0.6 | 13% | 2 |

Thus, as shown by example 2, also other methods than CRISPR/Cas9 may be used in the method according to the first aspect of the technology proposed herein to obtain the genetically modified plant cells according to the second aspect of the technology proposed herein.

### References

Andersson, M., Turesson, H., Nicolia, A., Fält, A.S., Samuelsson, M. & Hofvander, P. (2017). Efficient targeted multiallelic mutagenesis in tetraploid potato (Solanum tuberosum) by transient CRISPR-Cas9 expression in protoplasts. Plant Cell Reports, vol. 36 (1), pp. 117-128
Andersson, M., Turesson, H., Olsson, N., Fält, A.S., Ohlsson, P., Gonzalez, M.N., Samuelsson, M. & Hofvander, P. (2018). Genome editing in potato via CRISPR-Cas9 ribonucleoprotein delivery. Physiologia Plantarum, vol. 164 (4), pp. 378-384
Merino, I., Guasca, A.O., Krmela, A., Arif, U., Ali, A., Westerberg, E., Jalmi, S.K., Hajslova, J., Schulzova, V. & Sitbon, F. (2023). Metabolomic and transcriptomic analyses identify external conditions and key genes underlying high levels of toxic glycoalkaloids in tubers of stress-sensitive potato cultivars. Frontiers in Plant Science, vol. 14 (October), pp. 1-16
Nicolia, A., Fält, A.S., Hofvander, P. & Andersson, M. (2021). Protoplast-Based Method for Genome Editing in Tetraploid Potato. Methods Mol Biol. 2021;2264:177-186. doi: 10.1007/978-1-0716-1201-9_12.
Nicolia, A., Proux-wéra, E., Åhman, I., Onkokesung, N., Andersson, M., Andreasson, E. & Zhu, L. (2015). Targeted gene mutation in tetraploid potato through transient TALEN expression in protoplasts. Journal of Biotechnology, vol. 204, pp. 17-24 Elsevier B.V. DOI: https://doi.org/10.1016/j.jbiotec.2015.03.021
Petersson, E. V, Arif, U., Schulzova, V., Krtková, V., Hajšlová, J., Meijer, J., Andersson, H.C., Jonsson, L. & Sitbon, F. (2013b). Glycoalkaloid and calystegine levels in table potato cultivars subjected to wounding, light, and heat treatments. *Journal of agricultural and food chemistry,* vol. 61 (24), pp. 5893-5902 United States.
Simonovska, B. & Vovk, I. (2000). High-performance thin-layer chromatographic determination of potato glycoalkaloids. Journal of Chromatography A, vol. 903 (1-2), pp. 219-225

## Claims

1. A method of obtaining a genetically modified plant cell having reduced content of at least one steroidal glycoalkaloid, wherein the method comprises the steps of:
i. providing a plant cell, and
ii. modifying the genome of the plant cell so as to reduce or inhibit expression of at least one gene selected from the group consisting of *SMO1-L, DWF7-L,* and *TAM iso 2.*

2. The method according to claim 1, wherein the gene is selected from the group consisting of *SMO1-L* and *DWF7-L,* preferably wherein the gene is *SMO1-L.*

3. The method according to any preceding claim, wherein step ii comprises mutating or excising at least a part of said gene.

4. The method according to any preceding claim, wherein step ii is performed using CRISPR/Cas.

5. The method according to any preceding claim, wherein the genetically modified plant cell is from the family *Solanaceae,* preferably from the genus *Solanum,* more preferably from the species *Solanum tuberosum.*

6. A genetically modified plant cell having reduced content of at least one steroidal glycoalkaloid, wherein the genome of the plant cell has been modified so as to reduce or inhibit expression of at least one gene selected from the group consisting of *SMO1-L, DWF7-L,* and *TAM iso 2.*

7. The genetically modified plant cell according to claim 6, wherein the gene is selected from the group consisting of *SMO1-L and DWF7-L,* preferably wherein the gene is *SMO1-L.*

8. The genetically modified plant cell according to any of the claims 6-7, wherein the gene has been mutated or at least a part of the gene has been excised.

9. The genetically modified plant cell according to any of the claims 6-8, wherein the genome of the plant cell has been modified using CRISPR/Cas.

10. The genetically modified plant cell according to any of the claims 6-9, wherein the plant cell is from the family *Solanaceae,* preferably from the genus *Solanum,* more preferably from the species *Solanum tuberosum.*

11. A method of obtaining a genetically modified plant, comprising the steps of:
i. providing a genetically modified plant cell obtained by the method according to any of the claims 1-5, or according to any of the claims 6-10, and
ii. cultivating or regenerating the genetically modified plant cell to obtain the genetically modified plant.

12. A genetically modified plant comprising or consisting of:
- one or more genetically modified plant cells obtained by the method according to any of the claims 1-5, or
- one or more genetically modified plant cells according to any of the claims 6-10, or obtained by the method according to claim 11.

13. The genetically modified plant according to claim 12, wherein the plant is from the family *Solanaceae,* preferably from the genus *Solanum,* more preferably from the species *Solanum tuberosum.*

14. Use of a genetically modified plant according to any of the claims 12-13, or part thereof, preferably a tuber thereof, in a process for producing starch, wherein preferably at least one of amino acids, minerals, proteins, and dietary fibers is obtained and collected as a by-product of the process.

15. Use of a genetically modified plant according to any of the claims 12-13, preferably a part thereof, more preferably a tuber thereof, in a process for producing a food product, wherein preferably the food product is selected from the group consisting of peeled potato tubers, mashed potato or instant mashed potatoes, deep-fried potato products such as pommes frites or crisps, and potato flakes.
